(19) Europäisches Patentamt
European Patent Office
Office européen des brevets

(11) EP 3 920 877 B1

(12) **EUROPEAN PATENT SPECIFICATION**

(45) Date of publication and mention of the grant of the patent:
**17.12.2025 Bulletin 2025/51**

(21) Application number: **20701488.7**

(22) Date of filing: **29.01.2020**

(51) International Patent Classification (IPC):
***A61K 8/34*** *(2006.01)* ***A61K 8/41*** *(2006.01)*
***A61Q 5/00*** *(2006.01)* ***A61K 8/891*** *(2006.01)*
***A61K 8/898*** *(2006.01)*

(52) Cooperative Patent Classification (CPC):
**A61Q 5/00; A61K 8/416; A61K 8/891**

(86) International application number:
**PCT/EP2020/052117**

(87) International publication number:
**WO 2020/160970 (13.08.2020 Gazette 2020/33)**

(54) **COMPOSITION AND PROCESS FOR REDUCING THE DRYING TIME OF SOLID SUBSTRATES**

ZUSAMMENSETZUNG UND VERFAHREN ZUR VERRINGERUNG DER TROCKNUNGSZEIT VON FESTEN SUBSTRATEN

COMPOSITION ET PROCÉDÉ POUR RÉDUIRE LE TEMPS DE SÉCHAGE DE SUBSTRATS SOLIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.02.2019 EP 19156153**

(43) Date of publication of application:
**15.12.2021 Bulletin 2021/50**

(73) Proprietor: **Kao Corporation**
**Chuo-ku,**
**Tokyo 103-8210 (JP)**

(72) Inventors:
- **FUJINAGA, Hiroki**
  **Tokyo 131-0044 (JP)**
- **TETSU, Makio**
  **Tokyo 131-0044 (JP)**
- **PICKER, Andreas**
  **64297 Darmstadt (DE)**

(74) Representative: **Hoffmann Eitle**
**Patent- und Rechtsanwälte PartmbB**
**Arabellastraße 30**
**81925 München (DE)**

(56) References cited:
**EP-A1- 3 168 251**
**WO-A1-00/00174**
**WO-A1-2013/082096**
**WO-A2-95/05800**
**DE-A1- 102015 223 028**
**FR-A1- 3 059 900**
**US-A1- 2007 141 007**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 920 877 B1

## Description

### Field of the invention

The present invention relates to a composition, method, and use to reduce the drying time of solid substrates.

### Background of the invention

People in developed countries and newly industrialized countries alike experience a large amount of pollution in their everyday life stemming from incinerating fossil fuels generating electrical energy, propelling cars and aircrafts, and heating or cooling their homes. In addition, many industrial manufacturing processes produce polluted solid products, which cannot easily be sold to customers or used in subsequent production processes without prior cleansing treatment. Thus, industry and consumers face problems of cleansing solid substrates to remove pollutants. As many cleansing procedures require the application of aqueous compositions, drying the respective substrate before further use is highly desired. However, if air-temperature is low, air humidity high, or a speedy drying of the substrate is required, heat drying is a common technique to accelerate the process. In this respect, heat drying requires the application of energy, and, thus, must be as short as possible to reduce environmental burden.

A prominent illustration of such burden to cosmetic customers and environment has been published in a survey with more than 200 women and 200 teenagers over age 16 conducted in 2014 in the United States showing that women spend an average of 55 minutes each morning on their appearance [[1] https://www.today.com/health/how-we-did-today-aol-ideal-real-body-image-survey-2D12152771
[2] https://www.today.com/health/stop-obsessing-women-spend-2-weeks-year-their-appearance-today-2D12104866]. A not negligible part of this time is spent on the drying procedure of hair, especially for women with long hair. In light of this background it is not surprising that the cosmetic industry in general and hair care industry in particular attempts to find solutions to help long-hair customers accelerating their daily procedure and, thus, to save time. Most of the available products are so-called blow dry leave in sprays, based on lower alcohols solutions that help to evaporate water during the blow dry process. Some very few products are available in the rinse off area, promising reduced blow dry time and facilitating natural, non-alcohol-based evaporation. Unfortunately, these products are typically based on high concentrations of cyclopentasiloxanes (e.g. Mintel # 2303144), a silicone compound that quickly evaporates. However, this raw material is under regulatory discussion in some regions of the world.

When investigating the drying process of substrates such as hair, it starts prior to blow drying evaporation, in particular immediately after the final rinsing step of hair. The very first step is the drainage of water from the substrate. Moreover, this step is the decisive step for removing most of the water from the substrate. Thus, the more efficient the drainage in the drying process is prior to applying heat, the less time the customer/industry spends on later stage heat drying and the less energy has to be applied for drying.

In summary, it is paramount to research products that help to increase the velocity of water outflow for the convenience of consumers, efficiency of industry, and protection of the environment.

Inventors of the present invention have unexpectedly found out that a composition comprising a certain dialkyl quaternary ammonium cationic surfactants as well as low molecular weight dimethylpolysiloxanes at certain concentrations and weight ratios reduce the drying time of solid substrates, in particular of keratin fibers, enhance drainage of water from substrates, provide for a dry appearance, and a good bundle separation of keratin fibers.

Products comprising bis-(isostearoyl/oleoyl isopropyl) dimonium methosulfate and dimethicone are known from the prior art (e.g. Mintel # 5534485, # 3904857, # 5306377). However, the present invention utilizes low molecular weight dimethicones and certain weight ratios and concentrations of the compounds, and, thus, the invention is novel.

FR3059900 discloses bis-(isostearoyl/oleoyl isopropyl) dimonium methosulfate and amodimethicone, but the document is silent on low molecular weight dimethicones, and, thus, the invention is novel.

EP3168251 discloses bis-(isostearoyl/oleoyl isopropyl) dimonium methosulfate and dimethicone having a viscosity of 100 cs, and, therefore, a higher molecular weight as the dimethicones required by the present invention. Furthermore, it does not achieve the technical effect of the present invention as shown in the comparative examples.

DE102015222976 discloses bis-(isostearoyl/oleoyl isopropyl) dimonium methosulfate and high viscosity dimethicone, and, therefore, a higher molecular weight as the dimethicones required by the present invention. Furthermore, it does not achieve the technical effect of the present invention. Mention is also made of patent document WO 95/05800 which concerns topical personal care compositions containing polysiloxane-grafted adhesive polymers and having an improved drying time.

Despite the numerous attempts of the prior art, none of disclosures solve the problem of the present invention.

**Summary of the invention**

Thus, the first object of the present invention is a composition for reducing the drying time of solid substrates, preferably of keratin substrates, more preferably of human keratin substrates, further more preferably of human hair, comprising:

a) One or more quaternary ammonium surfactant(s) according to the following general structure

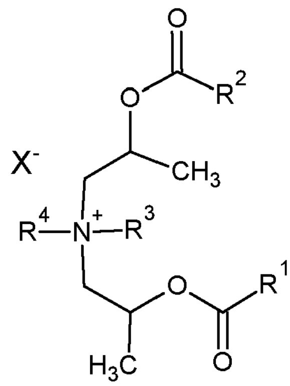

with the provision that $R^1$ and $R^2$ are $C_{11}$-$C_{21}$ linear or branched, saturated or unsaturated hydrocarbon groups, which may be the same or different and may be optionally substituted with one or more substituents selected from halogen, hydroxyl, amino, and $C_1$-$C_4$ alkoxy,

$R^3$ and $R^4$ are $C_1$-$C_3$ linear or branched hydrocarbon groups, which may be the same or different, and $X^-$ is selected from $Cl^-$, $Br^-$, $I^-$, sulfate, and methosulfate,

b) one or more dimethylpolysiloxane(s) having a weight average molecular weight $M_w$ in the range of 190 g/mol to 6000 g/mol determined by gel permeation chromatography,

wherein the total concentration of compounds according to a) is in the range of 0.1% to less than 5% by weight, calculated to the total weight of the composition, and

wherein the weight ratio of the compounds a) to b) is in the range of 0.075 to 8.

The second object of the present invention is a method for reducing the drying time of solid substrates, preferably of keratin substrates, more preferably of human keratin substrates, further more preferably of human hair comprising the steps of:

a) contacting the solid substrate with an aqueous composition,

b) applying the composition as defined above onto the substrate,

c) leaving the composition onto the substrate for a time period of 10 s to 600 s,

d) rinsing-off the solid substrate with water,

e) leaving the solid substrate for allowing drainage of water,

f) optionally drying the substrate with a heating device delivering an air temperature in the range of 35°C to 230°C.

It is preferred from the viewpoint of convenience for the user that between steps d) and f) no towel drying is performed.

However, in another aspect, it is preferred from the viewpoint of drying efficiency, time saving, and energy saving that towel drying is performed between steps d) and f).

It is further preferred from the viewpoint of user experience and routine that the composition of step a) is an

aqueous conditioning composition.

The third object of the present invention is a use of a composition as defined above for enhancing water drainage from solid substrates.

## Detailed description of the invention

The composition is suitable to reduce the drying time on various solid substrates such as wood, concrete, plastic surfaces, metal surfaces, and fiber surfaces. Suitable fiber surfaces are cotton, keratin fibers such as wool and hair. A suitable solid keratin substrate also is skin, preferably human skin. However, the present invention is particularly suitable for human keratin fibers, preferably human hair, from the viewpoint of its safety for human application.

### Compounds according to a)

Suitable compounds according to a) falling into the ambit of the structure according to claim 1 are displayed in Table 1.

Table 1: Suitable compounds according to a):

| Trade name | CAS/INCI | $R^1$ | $R^2$ | $R^3$ | $R^4$ |
|---|---|---|---|---|---|
| Varisoft EQ100* | Quaternium 98 | Iso-stearoyl | Oleoyl | $CH_3$ | $CH_3$ |
| n.a. | 1862231-90-0 | $C_{16}$ alkyl | $C_{16}$ alkyl | $CH_3$ | $CH_3$ |
| n.a. | 32208-07-4 | $C_{18}$ alkyl | $C_{18}$ alkyl | $CH_3$ | $CH_3$ |
| n.a. | 112065-30-2 | $C_{18}$ alkenyl | $C_{18}$ alkenyl | $CH_3$ | $CH_3$ |
| * Evonik Nutrition and Care GmbH | | | | | |

It is preferred from the viewpoint of biodegradability that the compound according to a) has the following general structure

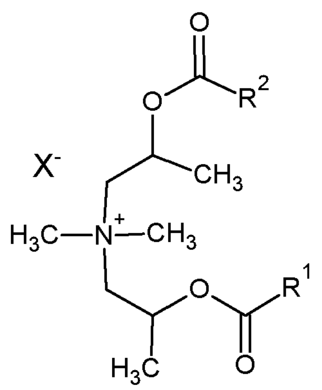

with the provision that $R^1$ and $R^2$ are $C_{11}$-$C_{21}$ linear or branched, saturated or unsaturated hydrocarbon groups, which may be the same or different and may be optionally substituted with one or more substituents selected from halogen, hydroxyl, amino, and $C_1$-$C_4$ alkoxy, and $X^-$ is selected from $Cl^-$, $Br^-$, $I^-$, sulfate, and methosulfate,

The most preferred compound according to a) is bis-(isostearoyl/oleoyl isopropyl) dimonium and/or its salt(s), from the viewpoint of commercial availability as well as biodegradability. Its suitable salts are $Cl^-$, $Br^-$, $I^-$, sulfate, and methosulfate.

The most preferred compound according to a) is bis-(isostearoyl/oleoyl isopropyl) dimonium methosulfate, from the viewpoint of commercial availability as well as biodegradability. The compound is available under the trade name Varisoft EQ100 from Evonik Nutrition and Care GmbH and has an INCI name registration application as Quaternium 98.

The total concentration of compounds according to a) is 0.1% by weight or more, preferably 0.2% by weight or more, more preferably 0.5% by weight or more, further more preferably 1% by weight or more, calculated to the total weight of the composition, from the viewpoint of water drainage, appearance and bundle separation.

The total concentration of compounds according to a) is less than 5% by weight, preferably 4% by weight or less, further more preferably 3% by weight or less, still further more preferably 2% by weight or less, calculated to the total weight of the composition, from the viewpoint of water drainage, appearance, and bundle separation.

For attaining the above-mentioned effects, the total concentration of compounds according to a) is in the range of 0.1% by weight to less than 5% by weight, preferably in the range of 0.2% by weight to 4% by weight, more preferably in the range of 0.5% by weight to 3% by weight, still more preferably in the range of 0.5% by weight to 2% by weight, still more preferably in the range of 1% by weight to 2% by weight, calculated to the total weight of the composition.

**Compounds according to b)**

For compounds according to b), weight average molecular weight needs to be determined. Molecular weight determination for measuring $M_w$ according to the present invention is suitably conducted by gel permeation chromatography.
The weight average molecular weight Mw of compound b) is determined by the method described below.
A sample is dissolved in chloroform at 25°C, so as to have a concentration of 0.5 g/100 mL. Using the following measurement apparatus and analytical column, chloroform is allowed to flow as an eluent at a rate of 1.0 ml per minute, and the column is stabilized in a thermostat at 40°C. One-hundred microliters of the sample solution is injected to the column to take the measurements. The detectors for detecting the compounds during chromatography are refractive index detectors The molecular weight of the sample is calculated on the basis of a calibration curve previously prepared. As the calibration curve of the molecular weight, one prepared by using several kinds of monodisperse polystyrenes as a standard sample is used.
Measurement Apparatus: HLC-8320GPC commercially available from Tosoh Corporation.
Analytical Column: K-804L + K-804L commercially available from SHOWA DENKO K.K.
Thus, the weight average molecular weight $M_w$ of compound b) is preferably 190 g/mol or more, more preferably 260 g/mol or more, further more preferably 320 mol/g or more, determined by gel permeation chromatography, from the viewpoint of water drainage and appearance.
The weight average molecular weight $M_w$ of compound b) is preferably 6000 g/mol or less, more preferably 5200 g/mol or less, further more preferably 2300 mol/g or less, still further more preferably 1300 mol/g or less, still further more preferably 760 mol/g or less, determined by gel permeation chromatography, from the viewpoint of water drainage, appearance and bundle separation.
For attaining the above-mentioned effects, the weight average molecular weight $M_w$ of compound b) is in the range of 190 g/mol to 6000 g/mol, more preferably in the range of 190 g/mol to 5200 g/mol, further more preferably in the range of 190 g/mol to 2300 g/mol, still further more preferably in the range of 260 g/mol to 1300 g/mol, still further more preferably in the range of 320 g/mol to 760 g/mol, determined by gel permeation chromatography.
The viscosity at 25°C of compound b) is preferably 0.65 $mm^2/s$ or more, more preferably 1 $mm^2/s$ or more, further more preferably 1.5 $mm^2/s$ or more, from the viewpoint of water drainage and appearance.
The viscosity at 25°C of compound b) is preferably 60 $mm^2/s$ or less, more preferably 50 $mm^2/s$ or less, further more preferably 20 $mm^2/s$, still further more preferably 10 $mm^2/s$ or less, still further more preferably 5 $mm^2/s$ or less, from the viewpoint of water drainage, appearance and bundle separation.
For attaining the above-mentioned effects, the viscosity at 25°C of compound b) is in the range of 0.65 $mm^2/s$ to 60 $mm^2/s$, more preferably in the range of 0.65 $mm^2/s$ to 50 $mm^2/s$, further more preferably in the range of 0.65 $mm^2/s$ to 20 $mm^2/s$ still further more preferably in the range of 1 $mm^2/s$ to 10 $mm^2/s$, still further more preferably in the range of 1.5 $mm^2/s$ to 5 $mm^2/s$.
The viscosity at 25°C of compound b) is measured with an Ubbelohde viscometer according to ASTM D 445-46 T or JIS Z 8803.
It is preferred that the total concentration of compounds according to b) is 0.2% by weight or more, preferably 0.5% by weight or more, further more preferably 1% by weight or more, still further more preferably 2% by weight or more, calculated to the total weight of the composition, from the viewpoint of water drainage, appearance, and bundle separation.
It is preferred that the total concentration of compounds according to b) is 20% by weight or less, preferably 15% by weight or less, more preferably 10% by weight or less, still more preferably 5% by weight or less, calculated to the total weight of the composition, from the viewpoint of appearance and cost of goods aspect.
For attaining the above mentioned effects, the total concentration of compounds according to b) is in the range of 0.2% to 20% by weight, preferably in the range of 0.2% to 15% by weight, more preferably in the range of 0.5% to 15% by weight, further more preferably in the range of 1% to 10% by weight, still further more preferably in the range of 2% to 5% by weight, calculated to the total weight of the composition.
Preferably, the weight ratio of the compounds a) to b) is in the range of 0.07 to 8, more preferably in the range of 0.15 to 3, more preferably in the range of 0.3 to 1, still further more preferably in the range of 0.4 to 0.8, from the viewpoint of water drainage, appearance, and bundle separation.

**Compounds according to c)**

In one aspect of the present invention, the composition further preferably comprises as compound c) one or more lipophilic compound(s) different from the compounds according to b), from the viewpoint of enhancing care level to keratin fibers.

Suitable one or more lipophilic compound(s) different from the compounds according to b) may preferably be selected from fatty alcohols having a branched or linear, saturated or unsaturated $C_{12}$ to $C_{22}$ alkyl chain, esters of alcohols having a linear or branched $C_3$ to $C_{18}$ alkyl chain with fatty acids having a linear or branched, saturated or unsaturated $C_{12}$ to $C_{22}$ alkyl chain, vegetable oils, and petrolatum-based products, and/or their mixtures, from the viewpoint of cosmetic safety and enhancing care level on keratin fibers.

It is further preferred that one or more lipophilic compound(s) different from the compounds according to b) is selected from fatty alcohols having branched or linear, saturated or unsaturated $C_{14}$ to $C_{18}$ alkyl chains, preferably saturated $C_{14}$ to $C_{18}$ alkyl chains, and/or their mixtures, more preferably it is a mixture of fatty alcohols having saturated and linear $C_{14}$ and $C_{16}$ alkyl chains, from the viewpoint of enhancing care level on keratin fibers, and good compatibility with hair conditioning products.

Suitable fatty alcohols having branched or linear, saturated or unsaturated $C_{12}$ to $C_{22}$ alkyl chains are, for example, lauryl alcohol, tridecyl alcohol, myristyl alcohol, pentadecyl alcohol, cetyl alcohol, palmitoleyl alcohol, heptadecyl alcohol, stearyl alcohol, oleyl alcohol, nonadecyl alcohol, arachidyl alcohol, behenyl alcohol, and/or their mixtures.

The preferred fatty alcohols are myristyl alcohol and cetyl alcohol, whereas the mixtures of the aforementioned two compounds is most preferred, from the viewpoint of enhancing care level on keratin fibers, and good compatibility with hair conditioning products.

Suitable esters of alcohols having a linear or branched $C_3$ to $C_{18}$ alkyl chain with fatty acids having a linear or branched, saturated or unsaturated $C_{12}$ to $C_{22}$ alkyl chain are isopropyl palmitate, isopropyl myristate, octyl palmitate, isocetyl palmitate, octyl stearate, oleyl oleate, ethylhexyl hydroxystearate, myristyl myristate, behenyl behenate, and/or their mixtures.

Suitable vegetable oils are jojoba oil, avocado oil, sunflower seed oil, walnut oil, peanut oil, olive oil, rapeseed oil, cottonseed oil, palm oil, sesame oil, soybean oil, coconut oil, safflower oil, sweet almond oil, macadamia nut oil, grapefruit seed oil, lemon kernel oil, orange kernel oil, apricot kernel oil, castor oil, argan oil, tamanu oil, grape seed oil, meadowfoam seed oil, black current seed oil, jasmine oil, bergamot fruit oil, basil oil, rice bran/germ oil, and/or their mixtures.

Suitable petrolatum-based products are liquid paraffins, especially paraffinum perliquidum and paraffinum subliquidum, and mineral oil, in particular white mineral oil, and/or their mixtures.

It is preferred that the total concentration of lipophilic compounds different from the compounds according to b) is 0.1% by weight or more, more preferably 0.5% by weight or more, further more preferably 0.75% by weight or more, calculated to the total weight of the composition, from the viewpoint of providing sufficient conditioning effect to keratin fibers.

It is preferred that the total concentration of lipophilic compounds different from the compounds according to b) is 10% by weight or less, more preferably 6% by weight or less, further more preferably 4% by weight or less, calculated to the total weight of the composition, from the viewpoint of cost of goods and stability of an aqueous composition.

Suitably, for attaining the above-mentioned effects, the total concentration of lipophilic compounds different from the compounds according to b) is in the range of 0.1% to 10% by weight, preferably 0.5% to 6% by weight, more preferably 0.75% to 4% by weight, calculated to the total weight of the composition.

It is preferred within this aspect of the present invention that the composition is an emulsion from the viewpoint of stability of the composition.

**Other surfactants different from a)**

For formulating an emulsion and from the viewpoint of further enhancing the stability of the composition, it may comprise one or more surfactant(s) different from a), preferably selected from cationic surfactants and/or non-ionic surfactants.

It is preferred from the viewpoint of enhancing composition stability and/or enhancing conditioning effect to keratin fibers that the total concentration of surfactants different from a) is in the range of 0.1% to 10% by weight, preferably in the range of 0.25% to 6% by weight, calculated to the total weight of the composition.

It is further preferred from the viewpoint of cosmetic acceptance of the composition that the pH of the composition is in the range of 3 to 8, preferably in the range of 3.0 to 7, more preferably in the range of 3.0 to 6.5, further more preferably in the range of 3.0 to 4.5, from the viewpoint of user application in a conditioning composition.

The following examples are to illustrate the present invention, but not to limit it.

## EXAMPLES

| | | | Working Examples | | | | | | | | | Comparative Examples | | |
|---|---|---|---|---|---|---|---|---|---|---|---|---|---|---|
| | | | 1 | 2 | 3 | 4 | 5 | 6 | 7 | 8 | 9 | 1 | 2 | 3 |
| | | | % by weight | | | | | | | | | | | |
| Composition | Compound a) | Quaternium-98 *1 | 1.5 | 1.5 | 1.5 | 1.5 | 1.5 | 1.5 | 1.5 | 0.2 | 0.5 | 1.5 | 1.5 | 5 |
| | Compound b) | DMPS (1 $mm^2/s$, $M_w$ 260)*2 | 2.5 | - | - | - | - | - | - | - | - | - | - | - |
| | | DMPS (2 $mm^2/s$, $M_w$ 392)*3 | - | 2.5 | - | 0.2 | 0.5 | 10 | 2.5 | 2.5 | 2.5 | - | - | 2.5 |
| | | DMPS (50 $mm^2/s$, $M_w$ 5130)*4 | - | - | 2.5 | - | - | - | - | - | - | - | - | - |
| | | DMPS (100 $mm^2/s$, $M_w$ 8870)*5 | - | - | - | - | - | - | - | - | - | - | 2.5 | - |
| | Lipophilic compound | Fatty alcohol ($C_{14}/_{16}$)*6 | - | - | - | - | - | - | 5 | - | - | - | - | - |
| | | NaOH/ HCl | Ad pH 3.7 ± 0.5 | | | | | | | | | | | |
| | | Water | Ad 100.0 | | | | | | | | | | | |
| | Ratio [(a/b)] | | 0.6 | 0.6 | 0.6 | 7.5 | 3 | 0.15 | 0.6 | 0.08 | 0.2 | | 0.6 | 2 |
| Evaluati | Water Drainage (%) | | 52.3 | 54.5 | 53.4 | 50.0 | 51.9 | 57.2 | 51.5 | 53.7 | 54.6 | 41.9 | 49.6 | 53.0 |
| | Appearance (△ $E_{ab}$) | | 9.0 | 8.0 | 10.2 | 10.5 | 9.1 | 10.0 | 9.2 | 8.8 | 8.7 | 14.4 | 12.1 | 16.2 |
| | Bundle Separation | | 9 | 9 | 8 | 8 | 9 | 9 | 8 | 8 | 8 | 5 | 5 | 3 |

*1 Varisoft EQ100 obtained from Evonik Nutrition and Care GmbH

*2-5 KF-96 1 cs – 100 cs series obtained from Shin-Etsu Chemical Co. Ltd., DMPS = dimethylpolysiloxane

*6 KALCOL 4098/KALCOL 6098 obtained from Kao Corp., mixing weight ratio 2 parts KALCOL 4098, 1 part KALCOL 6098

### Methods

### Preparation of hair streaks for water drainage measurements

Human hair fibers (Caucasian, 15 cm long) were purchased from Fischbach and Miller Haar, Laupheim, Germany. Hair fibers were merged to yield about 10 g hair per streak. The merged hair streaks were bleached twice with a bleaching solution and comprising 6% by weight ammonia (25% active) and 2% by weight ammonium chloride having a pH of 9.8, and 6% by weight hydrogen peroxide (50% active). The hair streaks were fully immersed with the bleaching solution in a water bath for 30 min per bleaching session. After completing the 2 bleaching treatments, the streaks were rinsed with water for 2 min, combed, and allowed to air-dry.

### Water drainage measurements

As a first step, dry weights of the merged hair streaks were determined. For this purpose, each streak was allowed to be wetted with water in a beaker for 15 min, then they were shampooed with a commercial shampoo available under the brand name Goldwell Dualsenses Scalp Specialist Deep Cleansing Shampoo, rinsed with lukewarm water for 1.5 min, and allowed to completely air-dry. Dry weights ($M_0$) were recorded.

For determination of wet weight ($M_1$) the hair streaks were allowed to be wetted for 12 min in a shaking bath filled with water. 5 g of the composition of the examples above was applied to each merged hair streak, the streaks were massaged for 1 min and the composition was allowed to rest for 2 min. The compositions were then rinsed off with lukewarm water for 1 min and water was allowed to drain for 2 min. Hair streaks were then centrifuged for 5 min 30 s (70 rpm, ~ 0.44 g) and after centrifugation the wet weights ($M_1$) of the streaks were determined. Water drainage (%) was then calculated according to the following equation:

$$\textit{Water drainage}\ (\%) = \left(1 - \frac{M_1 - M_0}{M_0}\right) \cdot 100\%$$

### Appearance measurements

Caucasian blond hair streaks were obtained from volunteers and merged as described above, but not bleached. The streaks were the shampooed with a commercially available shampoo under the brand name Goldwell Dualsenses Scalp Specialist Deep Cleansing Shampoo, thoroughly rinsed, and allowed to air-dry completely. After complete air-drying

of the hair streaks, dry hair color was measured by spectrophotometrical analysis with a Datacolor 45G CT instrument obtained from Datacolor Inc., Lawrenceville, NJ, USA. 5 measurements points on the hair streaks were averaged ($L_1$, $a_1$, $b_1$).

Hair streaks were then washed again with a commercially available shampoo under the brand name Goldwell Scalp Specialist Deep Cleansing Shampoo and rinsed-off with water. Then 3 g of the example compositions from above were applied onto the hair streaks, massaged for 1 min, and allowed to rest for 1 min. The streaks were then rinsed with lukewarm water for 1 min and water was drained by squeezing the hair streaks with the operator's finger. Then the streaks were blotted dry with a towel applying constant pressure onto the hair streaks (under use of a defined weight) for 30 s. The hair streaks were then combed through and color measurements of wet streaks were conducted at 5 different positions on the streak ($L_2$, $a_2$, $b_2$)
Based on the CIE*Lab color space results obtained by the measurements, $\triangle E_{ab}$ values for color difference were calculated according to the following equation:

$$\Delta E_{ab} = \sqrt{(L_2 - L_1)^2 + (a_2 - a_1)^2 + (b_2 - b_1)^2}$$

### Bundle separation measurements

Hair streaks were prepared as for appearance measurements. After complete air-drying, the streaks were immersed with 3 g of the example compositions, thoroughly rinsed with lukewarm water, and towel-dried. Bundle separation was then evaluated independently by 3 experts without providing details of the composition to the experts prior to their evaluation. The experts were asked to visually judge and rate the bundle separation according to the following criteria:

3: completely separated (many small bundles)
2: partially separated (some big and small bundles)
1: little separation (many big bundles)

The judgements of the experts were then added to yield a total score and are reported as integer numbers.

### Claims

**1.** A composition for reducing the drying time of solid substrates, preferably of keratin substrates, more preferably of human keratin substrates, further more preferably of human hair, comprising:

a) One or more quaternary ammonium surfactant(s) according to the following general structure

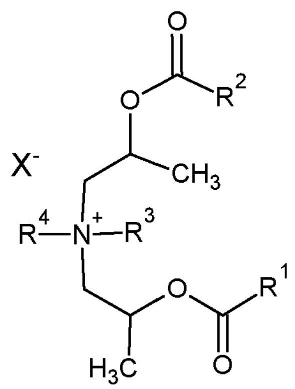

with the provision that $R^1$ and $R^2$ are $C_{11}$-$C_{21}$ linear or branched, saturated or unsaturated hydrocarbon groups, which may be the same or different and may be optionally substituted with one or more substituents selected from halogen, hydroxyl, amino, and $C_1$-$C_4$ alkoxy,
$R^3$ and $R^4$ are $C_1$-$C_3$ linear or branched hydrocarbon groups, which may be the same or different, and $X^-$ is selected from $Cl^-$, $Br^-$, $I^-$, sulfate, and methosulfate,

b) one or more dimethylpolysiloxane(s) having a weight average molecular weight $M_w$ in the range of 190 g/mol to 6000 g/mol determined by gel permeation chromatography,
wherein the total concentration of compounds according to a) is in the range of 0.1% to less than 5% by weight, calculated to the total weight of the composition, and
wherein the weight ratio of the compounds a) to b) is in the range of 0.075 to 8.

2. The composition according to claim 1 **characterized in that** the compound according to a) has the following general structure

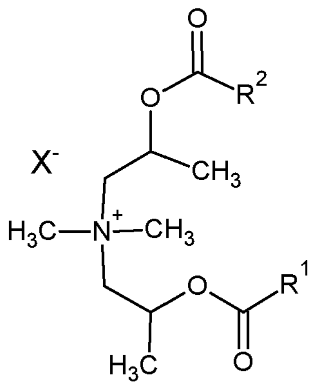

with the provision that $R^1$ and $R^2$ are $C_{11}$-$C_{21}$ linear or branched, saturated or unsaturated hydrocarbon groups, which may be the same or different and may be optionally substituted with one or more substituents selected from halogen, hydroxyl, amino, and $C_1$-$C_4$ alkoxy, and $X^-$ is selected from $Cl^-$, $Br^-$, $I^-$, sulfate, and methosulfate, preferably it is bis-(isostearoyl/oleoyl isopropyl) dimonium methosulfate.

3. The composition according to claims 1 and/or 2 **characterized in that** the total concentration of compounds according to a) is in the range of 0.1% by weight to less than 5% by weight, preferably in the range of 0.2% by weight to 4% by weight, more preferably in the range of 0.5% by weight to 3% by weight, still more preferably in the range of 0.5% by weight to 2% by weight, still more preferably in the range of 1% by weight to 2% by weight, calculated to the total weight of the composition.

4. The composition according to any of the preceding claims **characterized in that** the total concentration of compounds according to b) is in the range of 0.2% to 20% by weight, preferably in the range of 0.2% to 15% by weight, more preferably in the range of 0.5% to 15% by weight, further more preferably in the range of 1% to 10% by weight, still further more preferably in the range of 2% to 5% by weight, calculated to the total weight of the composition.

5. The composition according to any of the preceding claims **characterized in that** the weight ratio of the compounds a) to b) is in the range of 0.07 to .8, preferably in the range of 0.15 to 3, more preferably in the range of 0.3 to 1, still further more preferably in the range of 0.4 to 0.8.

6. The composition according to any of the preceding claims **characterized in that** the composition further comprises one or more lipophilic compound(s) as compound according to c) different from the compounds according to b).

7. The composition according to any of the preceding claims **characterized in that** it comprises one or more lipophilic compound(s) as compounds according to c) selected from fatty alcohols having a branched or linear, saturated or unsaturated $C_{12}$ to $C_{22}$ alkyl chain, esters of alcohols having a linear or branched $C_3$ to $C_{18}$ alkyl chain with fatty acids having a linear or branched, saturated or unsaturated $C_{12}$ to $C_{22}$ alkyl chain, vegetable oils, and petrolatum-based products, and/or their mixtures.

8. The composition according to any of the preceding claims **characterized in that** one or more lipophilic compound(s) as compound according to c) is selected from fatty alcohols having branched or linear, saturated or unsaturated $C_{14}$ to $C_{18}$ alkyl chains, preferably saturated $C_{14}$ to $C_{18}$ alkyl chains, and/or their mixtures, more preferably it is a mixture of fatty alcohols having saturated and linear $C_{14}$ and $C_{16}$ alkyl chains.

9. The composition according to any of the preceding claims **characterized in that** the total concentration of compounds

according to c) is in the range of 0.1% to 10% by weight, preferably 0.5% to 6% by weight, more preferably 0.75% to 4% by weight, calculated to the total weight of the composition.

**10.** The composition according to any of the preceding claims **characterized in that** the pH of the composition is in the range of 3 to 8, preferably in the range of 3.5 to 7, more preferably in the range of 3.5 to 6.5, further more preferably in the range of 3.5 to 4.5.

**11.** The composition according to any of the preceding claims **characterized in that** it comprises one or more surfactant(s) different from a), preferably selected from cationic surfactants and/or non-ionic surfactants.

**12.** The composition according to any of the preceding claims **characterized in that** it is an emulsion.

**13.** A method for reducing the drying time of solid substrates, preferably of keratin substrates, more preferably of human keratin substrates, further more preferably of human hair comprising the steps of:

a) contacting the solid substrate with an aqueous composition,
b) applying the composition according to any of the claims 1 to 12 onto the substrate,
c) leaving the composition onto the substrate for a time period of 10 s to 600 s,
d) rinsing-off the solid substrate with water,
e) leaving the solid substrate for allowing drainage of water,
f) optionally drying the substrate with a heating device delivering an air temperature in the range of 35°C to 230°C.

**14.** The method according to claim 13 **characterized in that** between steps d) and f) no towel drying is performed.

**15.** Use of a composition as defined in the claims 1 to 12 for enhancing water drainage from solid substrates.

**Patentansprüche**

**1.** Zusammensetzung zum Verkürzen der Trocknungszeit von festen Substraten, bevorzugt von Keratinsubstraten, mehr bevorzugt von menschlichen Keratinsubstraten, noch mehr bevorzugt von menschlichem Haar, umfassend:

a) ein oder mehrere quartäre Ammoniumtensid(e) gemäß der folgenden allgemeinen Struktur

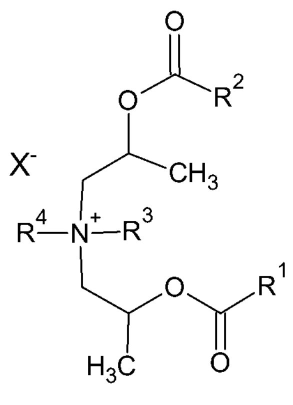

mit der Maßgabe, dass $R^1$ und $R^2$ lineare oder verzweigte, gesättigte oder ungesättigte $C_{11}$-$C_{21}$-Kohlenwasserstoffgruppen sind, die gleich oder verschieden sein können und optional mit einem oder mehreren Substituenten, ausgewählt aus Halogen, Hydroxyl, Amino und $C_1$-$C_4$-Alkoxy, substituiert sein können, $R^3$ und $R^4$ lineare oder verzweigte $C_1$-$C_3$-Kohlenwasserstoffgruppen sind, die gleich oder verschieden sein können, und $X^-$ aus $Cl^-$, $Br^-$, $I^-$, Sulfat und Methosulfat ausgewählt ist,

b) ein oder mehrere Dimethylpolysiloxan(e) mit einem gewichtsmittleren Molekulargewicht $M_w$ im Bereich von 190 g/Mol bis 6000 g/Mol, bestimmt durch Gelpermeationschromatographie,
wobei die Gesamtkonzentration der Verbindungen gemäß a) im Bereich von 0,1 Gew.-% bis weniger als 5 Gew.-%, berechnet auf das Gesamtgewicht der Zusammensetzung, liegt, und

wobei das Gewichtsverhältnis der Verbindungen a) zu b) im Bereich von 0,075 bis 8 liegt.

2. Zusammensetzung gemäß Anspruch 1, **dadurch gekennzeichnet, dass** die Verbindung gemäß a) die folgende allgemeine Struktur aufweist

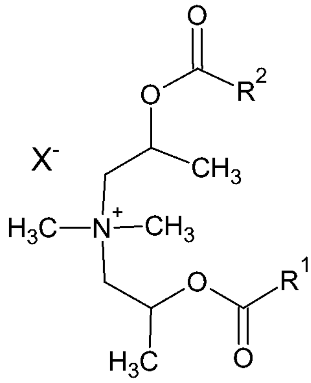

mit der Maßgabe, dass $R^1$ und $R^2$ lineare oder verzweigte, gesättigte oder ungesättigte $C_{11}$-$C_{21}$-Kohlenwasserstoffgruppen sind, die gleich oder verschieden sein können und optional mit einem oder mehreren Substituenten, ausgewählt aus Halogen, Hydroxyl, Amino, und $C_1$-$C_4$-Alkoxy, substituiert sein können, und $X^-$ aus $Cl^-$, $Br^-$, $I^-$, Sulfat und Methosulfat ausgewählt ist, es bevorzugt Bis-(isostearoyl/oleoylisopropyl)dimoniummethosulfat ist.

3. Zusammensetzung gemäß Anspruch 1 und/oder 2, **dadurch gekennzeichnet, dass** die Gesamtkonzentration der Verbindungen gemäß a) im Bereich von 0,1 Gew.-% bis weniger als 5 Gew.-%, bevorzugt im Bereich von 0,2 Gew.-% bis 4 Gew.-%, mehr bevorzugt im Bereich von 0,5 Gew.-% bis 3 Gew.-%, noch mehr bevorzugt im Bereich von 0,5 Gew.-% bis 2 Gew.-%, noch mehr bevorzugt im Bereich von 1 Gew.-% bis 2 Gew.-%, berechnet auf das Gesamtgewicht der Zusammensetzung, liegt.

4. Zusammensetzung gemäß einem der vorstehenden Ansprüche, **dadurch gekennzeichnet, dass** die Gesamtkonzentration der Verbindungen gemäß b) im Bereich von 0,2 Gew.-% bis 20 Gew.-%, bevorzugt im Bereich von 0,2 bis 15 Gew.-%, mehr bevorzugt im Bereich von 0,5 Gew.-% bis 15 Gew.-%, noch mehr bevorzugt im Bereich von 1 Gew.-% bis 10 Gew.-%, noch mehr bevorzugt im Bereich von 2 Gew.-% bis 5 Gew.-%, berechnet auf das Gesamtgewicht der Zusammensetzung, liegt.

5. Zusammensetzung gemäß einem der vorstehenden Ansprüche, **dadurch gekennzeichnet, dass** das Gewichtsverhältnis der Verbindungen a) zu b) im Bereich von 0,07 bis 8, bevorzugt im Bereich von 0,15 bis 3, mehr bevorzugt im Bereich von 0,3 bis 1, noch mehr bevorzugt im Bereich von 0,4 bis 0,8, liegt.

6. Zusammensetzung gemäß einem der vorstehenden Ansprüche, **dadurch gekennzeichnet, dass** die Zusammensetzung ferner eine oder mehrere lipophile Verbindung(en) als Verbindung gemäß c) umfasst, die sich von den Verbindungen gemäß b) unterscheiden.

7. Zusammensetzung gemäß einem der vorstehenden Ansprüche, **dadurch gekennzeichnet, dass** sie eine oder mehrere lipophile Verbindung(en) als Verbindungen gemäß c) umfasst, die aus Fettalkoholen mit einer verzweigten oder linearen, gesättigten oder ungesättigten $C_{12}$- bis $C_{22}$-Alkylkette, Estern von Alkoholen mit einer linearen oder verzweigten $C_3$- bis $C_{18}$-Alkylkette mit Fettsäuren mit einer linearen oder verzweigten, gesättigten oder ungesättigten $C_{12}$- bis $C_{22}$-Alkylkette, Pflanzenölen und Produkten auf Petrolatum-Basis und/oder deren Mischungen ausgewählt sind.

8. Zusammensetzung gemäß einem der vorstehenden Ansprüche, **dadurch gekennzeichnet, dass** eine oder mehrere lipophile Verbindung(en) als Verbindung gemäß c) aus Fettalkoholen mit verzweigten oder linearen, gesättigten oder ungesättigten $C_{14}$- bis $C_{18}$-Alkylketten, bevorzugt gesättigten $C_{14}$- bis $C_{18}$-Alkylketten, und/oder deren Mischungen ausgewählt ist, wobei es mehr bevorzugt eine Mischung von Fettalkoholen mit gesättigten und linearen $C_{14}$- und $C_{16}$-Alkylketten ist.

9. Zusammensetzung gemäß einem der vorstehenden Ansprüche, **dadurch gekennzeichnet, dass** die Gesamtkonzentration der Verbindungen gemäß c) im Bereich von 0,1 Gew.-% bis 10 Gew.-%, bevorzugt 0,5 Gew.-% bis 6 Gew.-%, mehr bevorzugt 0,75 Gew.-% bis 4 Gew.-%, berechnet auf das Gesamtgewicht der Zusammensetzung, liegt.

**10.** Zusammensetzung gemäß einem der vorstehenden Ansprüche, **dadurch gekennzeichnet, dass** der pH-Wert der Zusammensetzung im Bereich von 3 bis 8, bevorzugt im Bereich von 3,5 bis 7, mehr bevorzugt im Bereich von 3,5 bis 6,5, noch mehr bevorzugt im Bereich von 3,5 bis 4,5, liegt.

**11.** Zusammensetzung gemäß einem der vorstehenden Ansprüche, **dadurch gekennzeichnet, dass** sie ein oder mehrere Tensid(e) umfasst, die von a) verschieden sind, die bevorzugt aus kationischen Tensiden und/oder nichtionischen Tensiden ausgewählt sind.

**12.** Zusammensetzung gemäß einem der vorstehenden Ansprüche, **dadurch gekennzeichnet, dass** sie eine Emulsion ist.

**13.** Verfahren zum Verkürzen der Trocknungszeit von festen Substraten, bevorzugt von Keratinsubstraten, mehr bevorzugt von menschlichen Keratinsubstraten, noch mehr bevorzugt von menschlichem Haar, umfassend die Schritte:

a) das Inkontaktbringen des festen Substrats mit einer wässrigen Zusammensetzung,
b) das Aufbringen der Zusammensetzung gemäß einem der Ansprüche 1 bis 12 auf das Substrat,
c) das Belassen der Zusammensetzung auf dem Substrat für einen Zeitraum von 10 s bis 600 s,
d) das Abspülen des festen Substrats mit Wasser,
e) das Belassen des festen Substrats, um das Abfließen des Wassers zu ermöglichen,
f) optional das Trocknen des Substrats mit einer Heizvorrichtung, die eine Lufttemperatur im Bereich von 35°C bis 230°C liefert.

**14.** Verfahren gemäß Anspruch 13, **dadurch gekennzeichnet, dass** zwischen den Schritten d) und f) kein Trocknen mit einem Handtuch durchgeführt wird.

**15.** Verwendung einer Zusammensetzung, wie in den Ansprüchen 1 bis 12 definiert, zur Verbesserung des Ablaufens von Wasser von festen Substraten.

## Revendications

**1.** Composition pour réduire le temps de séchage de substrats solides, de préférence de substrats kératineux, plus préférentiellement de substrats kératineux humains, encore plus préférentiellement de cheveux humains, comprenant :

a) un ou plusieurs tensioactifs d'ammonium quaternaire selon la structure générale suivante

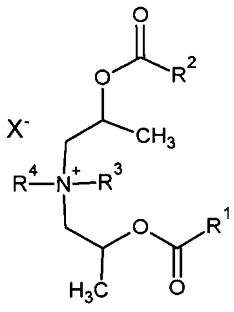

à condition que $R^1$ et $R^2$ soient des groupes hydrocarbonés en $C_{11}$ à $C_{21}$ linéaires ou ramifiés, saturés ou insaturés, qui peuvent être identiques ou différents et peuvent être facultativement substitués par un ou plusieurs substituants choisis parmi l'halogène, l'hydroxyle, l'amino et l'alcoxy en $C_1$ à $C_4$,
que $R^3$ et $R^4$ soient des groupes hydrocarbonés en $C_1$ à $C_3$, linéaires ou ramifiés, qui peuvent être identiques ou différents et que $X^-$ soit choisi parmi $Cl^-$, $Br^-$, $I^-$, le sulfate et le méthosulfate,

b) un ou plusieurs diméthylpolysiloxanes présentant une masse moléculaire moyenne en poids Mw dans la plage de 190 g/mol à 6000 g/mol, déterminée par chromatographie par perméation de gel,
dans laquelle la concentration totale en composés selon a) est dans la plage de 0,1 % à moins de 5 % en poids, calculée par rapport au poids total de la composition, et
dans laquelle le rapport pondéral composé a) sur composé b) est dans la plage de 0,075 à 8.

2. Composition selon la revendication 1, **caractérisée en ce que** le composé selon a) présente la structure générale suivante

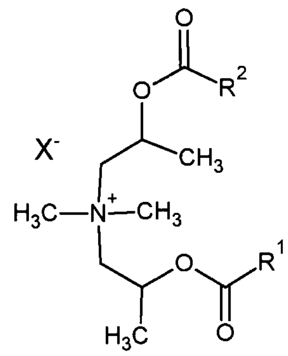

à condition que $R^1$ et $R^2$ soient des groupes hydrocarbonés en $C_{11}$ à $C_{21}$, linéaires ou ramifiés, saturés ou insaturés, qui peuvent être identiques ou différents et peuvent être facultativement substitués par un ou plusieurs substituants choisis parmi l'halogène, l'hydroxyle, l'amino et l'alcoxy en $C_1$ à $C_4$, et que $X^-$ soit choisi parmi $Cl^-$, $Br^-$, $I^-$, le sulfate et le méthosulfate, de préférence c'est le méthosulfate de bis-(isostéaroyl/oléoyl isopropyl)dimonium.

3. Composition selon les revendications 1 et/ou 2, **caractérisée en ce que** la concentration totale en composés selon a) est dans la plage de 0,1 % en poids à moins de 5 % en poids, de préférence dans la plage de 0,2 % en poids à 4 % en poids, plus préférentiellement dans la plage de 0,5 % en poids à 3 % en poids, encore plus préférentiellement dans la plage de 0,5 % en poids à 2 % en poids, et encore plus préférentiellement dans la plage de 1 % en poids à 2 % en poids, calculée par rapport au poids total de la composition.

4. Composition selon l'une quelconque des revendications précédentes **caractérisée en ce que** la concentration totale en composés selon b) est dans la plage de 0,2 % à 20 % en poids, de préférence dans la plage de 0,2 % à 15 % en poids, plus préférentiellement dans la plage de 0,5 % à 15 % en poids, encore plus préférentiellement dans la plage de 1 % à 10 % en poids, encore plus préférentiellement dans la plage de 2 % à 5 % en poids, calculée par rapport au poids total de la composition.

5. Composition selon l'une quelconque des revendications précédentes, **caractérisée en ce que** le rapport pondéral composé a) sur composé b) est dans la plage de 0,07 à 0,8, de préférence dans la plage de 0,15 à 3, plus préférentiellement dans la plage de 0,3 à 1, et encore plus préférentiellement dans la plage de 0,4 à 0,8.

6. Composition selon l'une quelconque des revendications précédentes, **caractérisée en ce que** la composition comprend en outre un ou plusieurs composés lipophiles en tant que composé selon c) différent des composés selon b).

7. Composition selon l'une quelconque des revendications précédentes, **caractérisée en ce qu'**elle comprend un ou plusieurs composés lipophiles en tant que composé selon c) choisis parmi les alcools gras présentant une chaîne alkyle en $C_{12}$ à $C_{22}$ linéaire ou ramifiée, saturée ou insaturée, les esters d'alcools présentant une chaîne alkyle linéaire ou ramifiée en $C_3$ à $C_{18}$ avec des acides gras présentant une chaîne alkyle en $C_{12}$ à $C_{22}$ linéaire ou ramifiée, saturée ou insaturée, les huiles végétales et les produits à base de pétrole, et/ou leurs mélanges.

8. Composition selon l'une quelconque des revendications précédentes, **caractérisée en ce qu'**un ou plusieurs composés lipophiles en tant que composé selon c) sont choisis parmi les alcools gras présentant des chaînes alkyle en $C_{14}$ à $C_{18}$ linéaires ou ramifiées, saturées ou insaturées, de préférence des chaînes alkyle saturées en $C_{14}$ à $C_{18}$, et/ou leurs mélanges, plus préférentiellement c'est un mélange d'alcools gras présentant des chaînes alkyle en $C_{14}$ et en $C_{16}$ saturées et linéaires.

9. Composition selon l'une quelconque des revendications précédentes, **caractérisée en ce que** la concentration totale en composés selon c) est dans la plage de 0,1 % à 10 % en poids, de préférence de 0,5 % à 6 % en poids, plus préférentiellement de 0,75 % à 4 % en poids, calculée par rapport au poids total de la composition.

10. Composition selon l'une quelconque des revendications précédentes, **caractérisée en ce que** le pH de la composition est dans la plage de 3 à 8, de préférence dans la plage de 3,5 à 7, plus préférentiellement dans la plage de 3,5 à 6,5, encore plus préférentiellement dans la plage de 3,5 à 4,5.

11. Composition selon l'une quelconque des revendications précédentes, **caractérisée en ce qu'**elle comprend un ou plusieurs tensioactifs différents de a), de préférence choisis parmi les tensioactifs cationiques et/ou les tensioactifs non ioniques.

12. Composition selon l'une quelconque des revendications précédentes, **caractérisée en ce qu'**il s'agit d'une émulsion.

13. Procédé pour réduire le temps de séchage de substrats solides, de préférence de substrats kératineux, plus préférentiellement de substrats kératineux humains, encore plus préférentiellement de cheveux humains, comprenant les étapes suivantes :

    a) mettre en contact le substrat solide avec une composition aqueuse,
    b) appliquer la composition selon l'une quelconque des revendications 1 à 12 sur le substrat,
    c) laisser la composition sur le substrat pendant une durée de 10 s à 600 s,
    d) rincer le substrat solide avec de l'eau,
    e) laisser le substrat solide pour permettre le drainage de l'eau,
    f) facultativement, sécher le substrat avec un dispositif chauffant fournissant une température d'air dans la plage de 35 °C à 230 °C.

14. Procédé selon la revendication 13, **caractérisé en ce qu'**aucun séchage à la serviette n'est effectué entre les étapes d) et f).

15. Utilisation d'une composition telle que définie dans les revendications 1 à 12 pour améliorer le drainage de l'eau des substrats solides.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 3059900
- EP 3168251 A
- DE 102015222976
- WO 9505800 A